# EUROPÄISCHE PATENTSCHRIFT

(11) **EP 1 051 604 B1**
(45) Veröffentlichungstag und Bekanntmachung des Hinweises auf die Patenterteilung: **08.05.2002**
(21) Anmeldenummer: 98908027.0
(22) Anmeldetag: 30.01.1998
(51) Int. Cl.: G01N 5/04, G01N 33/24

(54) **VERFAHREN ZUR BESTIMMUNG DER QUALITATIVEN ZUSAMMENSETZUNG DER ORGANISCHEN BODENSUBSTANZ VON MINERALBÖDEN**
METHOD FOR DETERMINING THE QUALITATIVE COMPOSITION OF THE ORGANIC SOIL SUBSTANCE OF MINERAL SOILS
PROCEDE POUR DETERMINER LA COMPOSITION QUALITATIVE DE LA SUBSTANCE ORGANIQUE DE SOLS MINERAUX

(43) Veröffentlichungstag der Anmeldung: 15.11.2000
(73) Patentinhaber: Siewert, Christian, 10318 Berlin (DE)
(72) Erfinder: Siewert, Christian, 10318 Berlin (DE)
(74) Vertreter: Ziebig, Marlene, Dr. Dipl.-Chem.
(86) Internationale Anmeldenummer: EP9800509
(87) Internationale Veröffentlichungsnummer: WO9939180

(56) Entgegenhaltungen:
- DD-A- 259 460
- DE-C- 19 638 731
- DATABASE WPI Section Ch, Week 8843 Derwent Publications Ltd., London, GB; Class J04, AN 88-305584 XP002077979 -& SU 1 386 899 A (SOIL INST) , 7. April 1988
- GAAL F ET AL: "Determination of the organic matter, metal carbonate and mobile water in soils. Simultaneous TG, DTG, DTA and EGA techniques" J THERM ANAL;JOURNAL OF THERMAL ANALYSIS NOV 1994 JOHN WILEY & SONS LTD, CHICHESTER, ENGL, Bd. 42, Nr. 5, November 1994, Seiten 1007-1016, XP002077975
- LEINWEBER PETER ET AL: "Organo-mineral soil clay fractions in fertilization experiments: Mineralogy, amounts and quality of organic matter and influence on soil properties" APPL CLAY SCI;APPLIED CLAY SCIENCE SEP 1993, Bd. 8, Nr. 4, September 1993, Seiten 295-311, XP002077976
- GOLEBIOWSKA D ET AL: "Correlation between derivatographic and chemiluminescence analysis data in relation to elemental composition of humic acids" PROCEEDINGS OF THE 1995 5TH NORDIC SYMPOSIUM ON HUMIC SUBSTANCES AND THE HUMOR/HUMEX PROJECT;LUND, SWEDEN JUN 6-8 1995, Bd. 22, Nr. 5, 6. Juni 1995, Seiten 495-500, XP002077977 Environ Int;Environment International 1996 Pergamon Press Inc, Tarrytown, NY, USA
- FRIEDRICH A ET AL: "Thermogravimetric and differential thermal analytical investigations on sewage farm soils" J THERM ANAL;JOURNAL OF THERMAL ANALYSIS JUN 1996 JOHN WILEY & SONS LTD, CHICHESTER, ENGL, Bd. 46, Nr. 6, Juni 1996, Seiten 1589-1597, XP002077978

## Beschreibung

Die Erfindung betrifft ein Verfahren zur Bestimmung der qualitativen Zusammensetzung der organischen Bodensubstanz (OBS) von Mineralböden mittels Thermogravimetrie. Das Verfahren ist einfach und kostengünstig und liefert für alle Mineralböden, unabhängig von Klimazonen, geologischen Ausgangssubstraten, der Bodengenese, der Nutzungsrichtung und anderen Einflüssen zuverlässige Werte.

Eines der wichtigsten Merkmale von Mineralböden ist die organische Bodensubstanz. Mit der organischen Bodensubstanz werden alle im Boden befindlichen organischen Verbindungen ohne lebende Biomasse bezeichnet. Die OBS setzt sich aus Humus, abgestorbener pflanzlicher und tierischer Biomasse, wasserlöslichen organischen Verbindungen (z.B. Kohlenhydrate, Aminosäuren, mikrobielle Exoenzyme, Lipide, pflanzliche und tierische Hormone u.a.) sowie anderen nichtlebenden organischen Substanzen oder organo-mineralischen Komponenten zusammen.

Der Begriff "Humus" kennzeichnet diejenigen organischen Bestandteile von Mineralböden, die sich während der Bodenbildung bzw. bei der Bodengenese anreichern. Sie unterscheiden sich von allen anderen organischen Substanzen durch ihre bodenspezifische Prägung.

Es ist eine große Vielfalt von klassischen Methoden insbesondere zur Analyse der humifizierten Komponenten der OBS bekannt. Sie sind auf eine Identifikation einzelner Komponenten mit physikalischen, chemischen oder biologischen Verfahren ausgerichtet, um Zusammenhänge der OBS zu einzelnen Bodeneigenschaften und insbesondere zur Bodenfruchtbarkeit aufzuklären.

Mit der Berücksichtigung ökologischer Aspekte in der bodenkundlichen Forschung erfolgte eine zunehmende Orientierung auf mikrobiell leicht abbaubare Komponenten der OBS mit dem Ziel, ökologische Probleme der industriellen Bodennutzung (z.B. Belastung der Gewässer mit Pflanzennährstoffen) z.B. durch verringerte Anwendung von Mineraldüngern und optimierte technologische Aufwendungen (z.B. Bodenbearbeitung) zu reduzieren, das Verhalten von Bodenschadstoffen zu beschreiben und andere Effekte (z.B. Versauerung von Waldböden) zu analysieren. Den humifizierten Bestandteilen der OBS wurde in diesem Zusammenhang als relativ stabilen Komponenten eine untergeordnete Bedeutung beigemessen.

Die damit verbundenen Entwicklungen von Methoden zur Qualitätsbewertung von Bestandteilen der OBS orientierten sich ebenfalls an einer Identifikation einzelner Stoffgruppen oder Verbindungen mit direktem Bezug zu aktuellen, regionalen Problemstellungen.

Hinweise auf allgemeingültige Eigenschaften, Funktionen oder Verhaltensweisen der OBS ließen sich aus diesen Methoden nicht ableiten. Aus diesem Grund sind bis heute keine Verfahren bekannt, mit denen die qualitative Zusammensetzung der OBS allgemeingültig bestimmt werden könnte.

In DD 249 972 A1 und DD 252 890 A1 werden beispielsweise Verfahren zur Bestimmung der Umsetzbarkeit der organischen Bodensubstanz beschrieben, bei denen die schwer mineralisierbaren Humussäuren abgetrennt werden, wodurch die leicht mineralisierbaren Bestandteile der OBS mit hoher Genauigkeit bestimmt werden können. Dies ermöglicht eine bessere Kalkulation der Nährstofffreisetzung aus dem Boden im Jahresverlauf. Beziehungen zu Klassifikationsmerkmalen von Böden, einzelnen Bodenbildungsprozessen oder Eigenschaften der Böden in einzelnen Klimazonen, Funktionen der Böden in der Biosphäre oder zu Gesetzmäßigkeiten der Sukzession von Ökosystemen lassen sich aus Ergebnissen dieser Methoden nicht ableiten.

DD 259 460 A1 enthält ein Verfahren, das die unterschiedliche Bedeutung der thermischen Stabilität humifizierter und nichthumifizierter Komponenten zur Bewertung der biologischen Zugänglichkeit und damit für die biologische Verwertbarkeit umsetzbarer Komponenten nutzt. Es ergänzt Verfahren zur quantitativen Bestimmung umsetzbarer Bestandteile durch einen qualitativen Parameter, bleibt aber auf einzelne Komponenten der OBS und davon abhängige Prozesse der Freisetzung von Pflanzennährstoffen beschränkt.

Ähnliche Schlußfolgerungen ergeben sich auch aus Untersuchungen zur thermischen Stabilität und biologischen Abbaubarkeit von Pflanzensubstanzen (Siewert, C. in Archiv für Acker- und Pflanzenbau und Bodenkunde, 1994, Bd. 38, S. 179-193). Die Ergebnisse verweisen auf eine Eignung der Thermogravimetrie zur halbquantitativen Erfassung biologisch umsetzbarer Anteile und anderer Merkmale (z. B. Hygroskopizität) in Ausgangsstoffen der organischen Bodensubstanz. Eine Übertragung der Schlußfolgerungen auf die organische Bodensubstanz oder eine Charakterisierung der qualitativen Zusammensetzung der organischen Bodensubstanz ist jedoch mit der beschriebenen Verfahrensweise nicht möglich.

Aus dem Fehlen einer allgemeingültigen Qualitätsbewertung der OBS resultieren zahlreiche Probleme der heutigen Bodenforschung. So erschweren gegenwärtig mehrere, miteinander nur begrenzt kompatible Klassifikationssysteme den weltweiten Vergleich und die einheitliche Interpretation von Ergebnissen zu Böden sowie die Beschreibung allgemeiner Funktionen von Böden und insbesondere der OBS in Ökosystemen.

Aufgabe der Erfindung war es deshalb, ein einfaches, kostengünstiges und für Mineralböden aller Regionen, Klimazonen und Nutzungsrichtungen anwendbares Verfahren zur Qualitätsbestimmung der organischen Bodensubstanz zur Verfügung zu stellen, welches die aufwendige stoffliche Identifizierung einzelner Komponenten der OBS nicht erforderlich macht und sich statt dessen an allgemeingültigen, evolutiv geprägten Gesetzmäßigkeiten der heutigen Bodenbildung orientiert.

Es wurde gefunden, daß die erfindungsgemäße Aufgabe durch ein einfaches und kostengünstiges thermogravimetrisches Verfahren gemäß Anspruch 1 gelöst werden kann.

Erfindungsgemäß wird die zu untersuchende lufttrockene Bodenprobe thermogravimetrisch analysiert, nachdem sie in einer dünnen Schicht bei konstanter und hoher relativer Luftfeuchte über 50% bis zur Gewichtskonstanz gelagert wurde. Insbesondere bevorzugt ist eine Lagerung bei 60-95% relativer Luftfeuchte. Die Lufttrocknung der Probe erfolgte vorzugsweise bei unter 30°C und über 50% relativer Luftfeuchte. Bei der thermogravimetrischen Analyse wird die Probe von 20°C auf über 960°C unter aeroben Bedingungen erwärmt, und die Gewichtsverluste aufgezeichnet. Die Erwärmungsgeschwindigkeit wird dabei zwischen 1°C und 50°C pro Minute so gewählt, daß eine schnelle, jedoch möglichst gleichzeitige Erwärmung des gesamten Probenvolumens erfolgt.

Ausgehend von der neuen Erkenntnis, daß Humusstoffe über eine Pufferung der biologischen Wirksamkeit mikrobieller Exoenzyme die Zusammensetzung der OBS bestimmen und diese Wirkung von klimatischen Faktoren sowie dem Tongehalt abhängig ist, läßt sich die Qualität der OBS über einen Quotienten aus den summaren thermogravimetrischen Gewichtsverlusten zwischen 200 °C und 450°C (vorrangiger Zerfallsbereich nichthumifizierter, mikrobiell umsetzbarer Komponenten der OBS) zu den summaren thermogravimetrischen Gewichtsverlusten zwischen 95 °C bis 190 °C (Gewichtsverluste durch Wasserabgabe) bestimmen. Vorzugsweise wird dieser erfindungsgemäße Gewichtsverlustquotient durch Division der Gewichtsverluste zwischen 295 °C und 305 °C durch die Gewichtsverluste zwischen 135 °C und 145 °C (vorrangiger Zerfallsbereich für gepufferte Exoenzyme) bestimmt. Dieser Gewichtsverlustquotient beschreibt Anteile an Nichthumusstoffen in der OBS bezogen auf die Menge gebundenen Wassers der Humusstoffe. Er ist unabhängig vom Tongehalt und gilt für alle Mineralböden unabhängig von der Region, der Klimazone oder der Nutzungsrichtung des Bodens.

Werden erfindungsgemäß Gewichtsverlustquotienten >1 gefunden, so handelt es sich um Böden, in denen durch Pufferwirkungen der Humusstoffe bei konstanter Bodenfeuchte mikrobiell umsetzbare Komponenten der OBS (Nichthumusstoffe) angereichert wurden und nur wenige oder keine gepufferten Exoenzyme vorliegen (z.B. Braunerden unter natürlicher Waldvegetation).

Werden erfindungsgemäß Gewichtsverlustquotienten <1 gefunden, so handelt es sich um Böden, deren Anteil an Exoenzymen größer ist als der Anteil an Nichthumusstoffen. Diese Charakteristika sind für Böden mit stark wechselnder Bodenfeuchte (z.B. Schwarzerden unter natürlicher Steppenvegetation) charakteristisch. Sie finden sich weiterhin in allen Bodenhorizonten mit bodengenetisch bedingt geringer Zufuhr an umsetzbaren organischen Rückständen bei sonst günstigen Lebensbedingungen für Bodenmikroorganismen (Horizonte unterhalb der A-Horizonte), d.h. in Schichten mit bodengenetisch bedingt geringen Anteilen an umsetzbaren Komponenten.

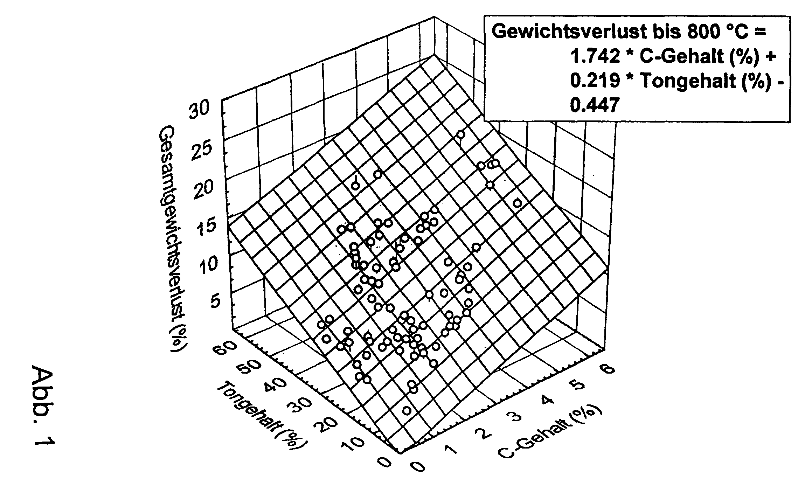

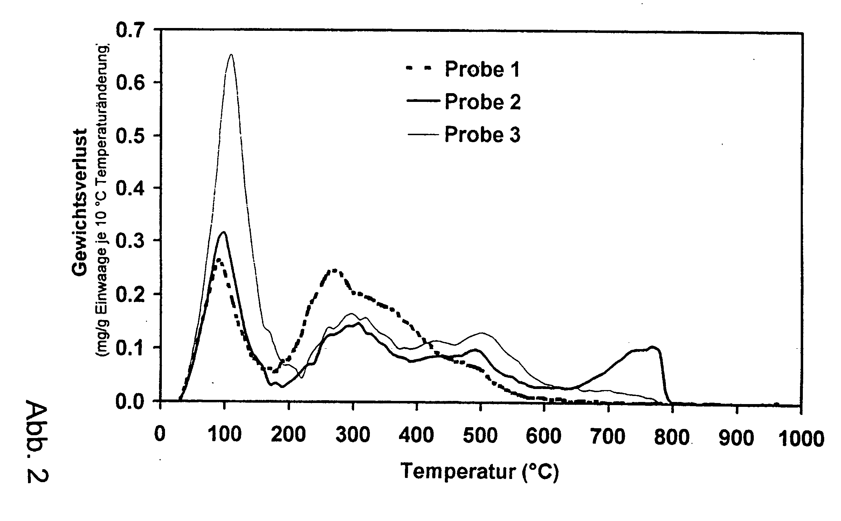

Unter Ausnutzung der engen Beziehungen zwischen den thermogravimetrischen Gewichtsverlusten bei Erwärmung von 20 °C auf über 960 °C (vorzugsweise von 20 °C auf 800 °C) vom Tongehalt und vom Gehalt an organischem Kohlenstoff (s. Abb. 1), sowie der Erfassung von Gewichtsanteilen humifizierter organischer Bestandteile der OBS mit den tonabhängigen Gewichtsverlusten läßt sich auch der Gewichtsanteil umsetzbarer Komponenten der OBS quantifizieren. Er errechnet sich aus dem Gesamtgewichtsverlust abzüglich der Gewichtsverluste tonabhängiger Komponenten der OBS unter Verwendung des in Abb. 1 dargestellten Koeffizienten für den Einfluß des Tongehaltes auf den Gesamtgewichtsverlust sowie abzüglich der Gewichtsverluste durch Karbonate.

Um die Gewichtsverluste umsetzbarer organischer Substanz in die Menge umsetzbaren Kohlenstoffs umzurechnen läßt sich ein Faktor bestimmen, welcher den mittleren Gewichtsverlust der OBS je % C im Boden ausdrückt. Er errechnet sich aus der Division des Gesamtgewichtsverlustes bis 800 °C abzüglich der Gewichtsverluste durch hygroskopisches Wasser durch den C-Gehalt des Bodens in Prozent. Die Division der Gewichtsverluste der umsetzbaren Substanz durch diesen Faktor liefert Informationen über den Gehalt an umsetzbaren Kohlenstoff in Prozent im Boden.

Durch die Bestimmung des erfindungsgemäßen Gewichtsverlustquotienten ist es in Kombination mit der Bestimmung umsetzbarer OBS-Anteile möglich, Spezifika der Nährstofffreisetzung für die produktive Bodennutzung (Forst- und Landwirtschaft) und Aufgaben des Bodenschutzes gezielt zu nutzen. Derartige Möglichkeiten werden im Folgenden an Einzelbeispielen erläutert.

Lassen sich beispielsweise in zwei benachbarten Böden unter gleichen bodenklimatischen Bedingungen gleiche Gewichtsverlustquotienten nachweisen, verhält sich die Menge umsetzbarer Substanzen proportional zur Menge von Pflanzennährstoffen, die durch biologische Umsatzprozesse freigesetzt werden können. Liefert hingegen die Bestimmung der Menge umsetzbarer Substanzen gleiche Werte und ist der Gewichtsverlustquotient verschieden, muß bei dem Boden mit höherem Gewichtsverlustquotient mit einer langsameren Freisetzung von Nährstoffen in Folge einer im Mittel gleichmäßigeren Bodenfeuchte gerechnet werden.

Veränderungen des Gewichtsverlustquotienten im Jahresverlauf weisen auf witterungsbedingte Veränderungen der OBS - Qualität hin. Sinkt beispielsweise der Gewichtsverlustquotient während der Sommermonate in Folge wechselnder Bodenfeuchte bei gleichbleibendem Gehalt an umsetzbarer Substanz, ist dies ein Anzeichen für die Zunahme des Anteils gepufferter Exoenzyme in der OBS. Letztere führen bei Zunahme der Bodenfeuchte im Herbst oder in den Wintermonaten zu einer hohen Freisetzung von Nährstoffen, die von überwinternden Kulturen genutzt werden können oder aber das Grundwasser durch Auswaschung belasten. Nach gleichmäßig feuchten Sommern bzw. bei nur geringfügigem Absinken der Gewichtsverlustquotienten ist hingegen mit einer Nährstofffreisetzung in der kalten Jahreszeit oder bei Zunahme der Bodenfeuchte nicht zu rechnen, so daß überwinternde Kulturen im Interesse der Ertragsgestaltung mit geringeren Risiken für die Verlagerung von Nährstoffen gedüngt werden können.

Der Gewichtsverlustquotient variiert am stärksten in Abhängigkeit vom Klima und dem Einfluß von Grundwasser auf die Bodenfeuchte, weniger in Abhängigkeit von der Vegetation, dem Tongehalt und anderen Faktoren sowie am geringsten im Jahresverlauf. Dies ermöglicht bei bekannten bodenklimatischen Bedingungen (Klima, Grundwassereinfluß, Vegetation) eine Diagnostik von Bodentypen an Hand des Gewichtsverlustquotienten bzw. der sich darin ausdrückenden OBS - Qualität. Bei landwirtschaftlichen Böden ist eine Bewertung des Versorgungszustandes mit organischer Substanz möglich. Ist hingegen der Bodentyp, die Bodennutzung und Zufuhr an umsetzbaren organischen Rückständen bekannt, erlaubt der Gewichtsverlustquotient Rückschlüsse über Spezifika der Bodenbildung, insbesondere zur mittleren Variabilität der Feuchtebedingungen.

Desweiteren sind Schlußfolgerungen auf ökologische Eignung eines Bodens für unterschiedliche Nutzungsrichtungen möglich. So weisen beispielsweise hohe Gewichtsverlustquotienten auf konstante Feuchteverhältnisse und eine langsame Nährstofffreisetzung hin, die in den meisten Fällen der Entzugsdynamik der Waldvegetation besser entspricht. Sehr kleine Gewichtsverlustquotienten weisen im Gegensatz dazu insbesondere in Kombination mit hohen Absolutwerten für den Gehalt an umsetzbarer Substanz auf eine Eignung des Bodens für landwirtschaftliche Kulturen mit kurzer, steppenähnlicher Vegetationsperiode (z.B. die meisten Getreidearten) hin.

Hinsichtlich der Klassifikation von Böden weisen kleine Gewichtsverlustquotienten auf wechselfeuchte Bedingungen hin, wie sie z.B. für die Aₕ - Horizonte (Humusanreicherungshorizonte) von Podsolen und Schwarzerden charakteristisch sind. Große Gewichtsverlustquotienten sind hingegen unter anderem für Braunerden und Böden der gleichmäßig feuchten Tropen (unter natürlicher Waldvegetation) typisch. Unterschiede im Gewichtsverlustquotienten werden zugleich von Unterschieden in der Intensität der Färbung (Farbintensität der OBS je C - Gehalt) begleitet, die sich für eine vergleichende Diagnostik der OBS - Qualität verwenden lassen, wenn gleiche Gehalte an färbenden Kationen (z.B. Fe, Mn) vorliegen. Hieraus ergeben sich Möglichkeiten für die Entwicklung von Feldmethoden, welche durch das thermogravimetrische Verfahren regional geeicht und angepaßt werden können.

Nachfolgend soll die Erfindung anhand von Ausführungsbeispielen näher beschrieben werden, ohne sie darauf einzuschränken.

### Ausführungsbeispiele

Analysiert wurden folgende Proben:
1. Parabraunerde aus der Hildesheimer Börde auf Löß unter altem Buchenmischwald
2. Parabraunerde aus der Hildesheimer Börde auf Löß unter landwirtschaftlicher Nutzung unmittelbar neben Probe 1 gelegen (gleiche geologische und klimatische Ausgangsbedingungen)
3. Braunschwarzerde aus Keupermaterial unter landwirtschaftlicher Nutzung aus der Nähe von Weimar

Die thermogravimetrischen Analysen dieser Proben wurden mit einem umgebauten "Derivatographen" der ungarischen Firma "MOM" durchgeführt und die Ergebnisse durch gleiche Analysen mit einer Thermowaage der Mettler-Toledo GmbH (TGA/SDTA 851^{e}) verifiziert. Die Erwärmung erfolgte von Zimmertemperatur (20 - 25 °C) auf über 960 °C mit einer Aufheizrate von 5 °C pro Minute unter aeroben Bedingungen. Während der thermogravimetrischen Analyse wurden die Temperatur und der Gewichtsverlust kontinuierlich aufgezeichnet. Die Einwaage der Bodenproben betrug 1700 mg (bei der TGA/SDTA 851^{e}:900 mg). Zur Vorbereitung der Analyse wurden die lufttrockenen Bodenproben in einer max. 3 mm dicken Schicht bei 76 % relativer Luftfeuchte über 10 Tage gelagert, um eine Einstellung gleicher Feuchteverhältnisse in allen Proben zu garantieren.

**Tabelle 1**

| Ausgewählte Meßwerte der thermogravimetrischen Analyse | | | | | | | | |
|---|---|---|---|---|---|---|---|---|
| summare Gewichtsverluste in mg / g Einwaage für die Temperaturbereiche | | | | | | | | |
| von (°C) | 295 | 135 | 30 | 105 | 355 | 415 | 660 | 30 |
| bis (°C) | 305 | 145 | 800 | 115 | 365 | 425 | 970 | 190 |
| Spalte | 1 | 2 | 3 | 4 | 5 | 6 | 7 | 8 |
| Probe 1 | 0.205 | 0.088 | 6.90 | 0.195 | 0.175 | 0.107 | 0.16 | 2.05 |
| Probe 2 | 0.143 | 0.098 | 6.77 | 0.256 | 0.092 | 0.085 | 0.97 | 2.27 |
| Probe 3 | 0.165 | 0.307 | 9.38 | 0.653 | 0.116 | 0.113 | 0.40 | 4.54 |

Aufbauend auf Tab. 1 wurde die Qualität der OBS durch Bildung des Quotienten der Gewichtsverluste zwischen 295 °C und 305 °C (Spalte 2) zu den Gewichtsverlusten zwischen 135 °C und 145 °C (Spalte 3) für jede Probe ermittelt. Dabei ergab sich für Probe 1 folgende Berechnung: 0.205 / 0.088 = 2.33. Analog wurde für die Proben 2 und 3 verfahren, womit sich für Probe 2 ein OBS-Qualitätsindex von 1,46 und für Probe 3 von 0,54 ergibt.

Die Werte zur OBS - Qualität von Probe 1 dienen hier als Vergleichsgrundlage. Sie sind für Aₕ - Horizonte (Humusanreicherungshorizonte) von Böden des gemäßigten humiden Klimas unter Wald bzw. von Braunerden unter natürlicher Vegetation typisch. Die landwirtschaftliche Nutzung des gleichen Bodens (Probe 2) führt zu einer deutlichen Reduktion des umsetzbaren Kohlenstoffs, während der Gehalt des inerten bzw. humifizierten Kohlenstoffs sich nicht verändert (Differenz aus C-Gehalt und C der umsetzbaren Substanz). Hieraus erklärt sich ein deutlich geringerer Gewichtsverlustquotient für die OBS - Qualität. Letzterer ist bei Probe 3 nochmals deutlich kleiner und erreicht Größenordnungen von Schwarzerden, d.h. von Böden mit ausgeprägter Wechselfeuchte und Steppenvegetation. Ursache dieses für humide Klimaregionen kleinen Gewichtsverlustquotienten bei Probe 3 ist neben der landwirtschaftlichen Nutzung eine deutlich erhöhte Variabilität der Bodenfeuchte, die aus niedrigeren Niederschlägen in der betreffenden Region und aus einer geringeren Wasserhaltefähigkeit des tonreichen Bodens im Vergleich zu den Proben 1 und 2 auf Löß resultiert.

Für die Bodennutzung läßt sich aus diesen Ergebnissen zum Beispiel eine bessere Eignung der Braunschwarzerde für die landwirtschaftliche Produktion bzw. insbesondere für Kulturen mit kurzer Vegetationsperiode (ursprüngliche Steppenpflanzen) ableiten, weil - im Unterschied zur Probe 2 - bei Zufuhr von organischer Substanz mit einer schnelleren Freisetzung von Nährstoffen zu rechnen ist. Allerdings liegt der Anteil an umsetzbarer Substanz deutlich unter dem normalen Niveau von Schwarzerden (nicht dargestellt). Die Ausnutzung des Ertragspotentials der Probe 3 erfordert demnach eine deutlich höhere Zufuhr an organischer Substanz als bisher praktiziert, d.h. im Vergleich zur Probe 2 ist das Defizit an organischen Düngestoffen nutzungsbedingt beträchtlich höher (hier eine Konsequenz der über Jahrzehnte intensiveren Ackernutzung).

Probe 1 bietet demgegenüber durch die ausgeprägte Pufferung von Nährstoffressourcen die besten Voraussetzungen für eine langsame und andauernde Nährstoffnachlieferung, wie sie für forstwirtschaftliche Belange und die Reinhaltung des Grundwassers (Reduktion der Verlagerung von Nährstoffen) günstig ist.

Desweiteren läßt sich der Gewichtsverlustquotient als Klassifikationsmerkmal verwerten, weil Werte über 1 im Ap - Horizont (Pflughorizont, Proben 1 und 2) ausschließlich bei Braunerden und nur in seltenen Fällen bei lessivierten (tondurchschlämmten) und podsolierten Böden (Böden mit Tonzerstörung) auftreten. Die Ergebnisse der Qualitätsbewertung bestätigen insofern die Richtigkeit der Zuordnungen der Proben zu einzelnen Bodentypen und zu Klimazonen (letzteres nur bei bekannter Vegetation), auch ohne Kenntnisse zum Profilaufbau. Der niedrige Gewichtsverlustquotient bei Probe 3 verweist hingegen auf eine Zuordnung zu Schwarzerden. Ohne die Einbeziehung weiterer Proben läßt sich jedoch nicht sagen, welcher Qualitätsquotient für diesen Boden unter natürlicher Vegetation charakteristisch ist. Es lassen sich jedoch wegen der festgestellten Unterversorgung mit organischer Substanz Werte deutlich näher 1 erwarten, so daß es sich nur um einen Übergangsboden von Schwarzerden zu Braunerden handeln kann. Diese Schlußfolgerung stimmt mit der an Hand des Profilaufbaus getroffen Zuordnung des Bodens zu Braunschwarzerden überein. Auch bei Probe 3 ist somit an Hand der Qualitätsbewertung bei Kenntnis der ackerbaulichen Nutzung und der wichtigsten klimatischen Bodenbildungsbedingungen eine Einschätzung des Bodentyps möglich.

## Patentansprüche

1. Verfahren zur Bestimmung der qualitativen Zusammensetzung der organischen Bodensubstanz (OBS) von Mineralböden,
**dadurch gekennzeichnet,**
**daß** eine gewichtskonstante Bodenprobe in üblicher Weise thermogravimetrisch analysiert wird und der Quotient aus den summaren Gewichtsverlusten der Probe bei Erwärmung von 200 bis 450°C zu den summaren Gewichtsverlusten der Probe bei Erwärmung von 95 bis 190°C gebildet wird.

2. Verfahren nach Anspruch 1,
**dadurch gekennzeichnet, daß**
der Quotient aus den summaren Gewichtsverlusten der Probe bei Erwärmung von 295 auf 305°C zu den summaren Gewichtsverlusten der Probe bei Erwärmung von 135°C auf 145°C gebildet wird.

## Claims

1. A method of determining the qualitative composition of soil organic matter (SOM) of mineral soils, **characterized by** subjecting a soil sample of constant weight to thermogravimetric analysis in the usual way and forming the quotient of the overall weight loss of the sample during heating from 200°C to 450°C and the overall weight loss of the sample during heating from 95°C to 190°C.

2. The method according to claim 1, **characterized by** forming the quotient of the overall weight loss of the sample during heating from 295°C to 305°C and the overall weight loss of the sample during heating from 135°C to 145°C.

## Revendications

1. Procédé d'analyse de la composition qualitative de la substance organique du sol (SOS) contenue dans les sols minéraux,
**caractérisé en ce**
**que** l'on analyse un échantillon de sol d'un poids constant par thermogravimétrie de manière habituelle et que l'on détermine le quotient obtenu en divisant la perte de poids totale de l'échantillon qui se produit en chauffant de 200° C à 450° C par la perte totale de poids de l'échantillon qui se produit en chauffant de 95° C à 190°C.

2. Procédé d'après la revendication 1,
**caractérisée en ce**
**que** l'on détermine le quotient obtenu en divisant la perte de poids totale de l'échantillon qui se produit en chauffant de 295° C à 305° C par la perte totale de poids de l'échantillon qui se produit en chauffant de 135° C à 145° C.
